# EUROPEAN PATENT SPECIFICATION

(11) **EP 3 366 292 B1**
(45) Date of publication and mention of the grant of the patent: **02.12.2020**
(21) Application number: 15900611.3
(22) Date of filing: 13.10.2015
(51) Int. Cl.: A61K 31/385, A61K 31/195, A23L 33/10, A61P 3/04, A61K 9/00, A61K 9/08, A61K 9/14, A61K 9/20, A61K 9/48, A61K 31/198, A23L 33/12, A23L 33/175, A61P 3/00

(54) **COMPOSITION FOR PREVENTING OR TREATING OBESITY CONTAINING ALPHA-LIPOIC ACID AND N-ACETYLCYSTEINE AS ACTIVE INGREDIENTS**
ZUSAMMENSETZUNG ZUR VORBEUGUNG ODER BEHANDLUNG VON ADIPOSITAS MIT ALPHA-LIPONSÄURE UND N-ACETYLCYSTEIN ALS WIRKSTOFFE
COMPOSITION POUR LA PRÉVENTION OU LE TRAITEMENT DE L'OBÉSITÉ CONTENANT DE L'ACIDE ALPHA-LIPOÏQUE ET DE LA N-ACÉTYLCYSTÉINE EN TANT QUE SUBSTANCES ACTIVES

(43) Date of publication of application: 29.08.2018
(73) Proprietor: The Asan Foundation, Seoul 05505 (KR)
(72) Inventor: LEE, Ki Up, Yangpyeong-gun Gyeonggi-do 12580 (KR); PARK, Joong Yeol, Guri-si Gyeonggi-do 11948 (KR); KOH, Eun Hee, Seoul 05507 (KR); JANG, Jung Eun, Seoul 05515 (KR); LEE, In Kyu, Daegu 42106 (KR)
(74) Representative: Vossius & Partner Patentanwälte Rechtsanwälte mbB
(86) International application number: PCT/KR2015/010783
(87) International publication number: WO 2017/065328

(56) References cited:
- KR-A- 20020 092 640
- KR-A- 20040 094 528
- KR-A- 20090 075 269
- US-A1- 2006 263 446
- US-A1- 2014 045 874
- US-A1- 2014 243 400
- KIM, M. -S. ET AL.: 'Anti-obesity Effects of alpha-lipoic Acid Mediated by Suppression of Hypothalamic AMP-activated Protein Kinase' NATURE MEDICINE vol. 10, no. 7, 2004, pages 727 - 73, XP055375693
- KANG, SEOK WOO ET AL.: 'The Effect or Acid on Proteinuria and Renal TGF Expression in Obese Type 2 Diabetic Rat Model' KOREAN DIABETES ASSOCIATION vol. 32, 2008, pages 21 - 29, XP055375696

## Description

### [Technical Field]

The present invention relates to a composition for preventing or treating obesity, containing α-lipoic acid and N-acetylcysteine as active ingredients.

### [Background Art]

Obesity is a state where excess fat is accumulated in the body. The world health organization (WHO) has reported an increase in obesity worldwide, and assumed that an higher intake of foods having high energy density with low contents of vitamins, minerals, and other trace nutrients, and lower physical activity is the reason for the increase in obesity. Thus, the basic cause of obesity is considered as an imbalance between the enegic intake and energy consumption. Obesity also increases the risk of lifestyle-related diseases, such as type 2 diabetes and arteriosclerosis, fatty liver, cholelithiasis, and hepatic steatosis that leads to liver cirrhosis. Further, an excessive weight gain has a bad effect on bones and joints to cause skeletal abnormalities and limit the activity. As a result, a vicious cycle in which obesity is further intensified occurs.

As such, obesity induces many metabolic diseases, and the development of easy-to-use therapeutic agents is urgently needed.

Until now, obesity therapeutic agents are largely classified into an appetite suppressant, a fat absorption inhibitor, and an energy consumption promoter. Rrepresentative obesity therapeutic drugs which are licensed for long-term use include orlistat (trade name: XENICAL) and sibutramine (trade name: REDUCTIL). The orlistat as a lipase inhibitor inhibits lipase secreted from the pancreas and the digestive system as an inhibitor of lipase secreted from the pancreas and the digestive system to prevent absorption of fat, and the sibutramine as an appetite suppressant inhibits resorption of norepinephrine and serotonin. However, the orlistat causes reduced absorption of fat-soluble vitamins, abdominal discomfort, gastric bloating, greasy stool, and the like and the sibutramine causes side effects such as headache, dry mouth (severe thirst), insomnia, constipation, and the like. Accordingly, recently, obesity therapeutic agents without side effects have highlighted.

Meanwhile, α-lipoic acid (C₈H₁₄O₂S₂) has various names such as thioctic acid, 6,8-dithioctanoic acid, 1,2-diethylene-3-pentanoic acid, and the like. As it is known that an unknown trace ingredient contained in the liver and yeast extracts is a growth factor of lactic acid bacteria, the α-lipoic acid was introduced as a new vitamin. In 1951, L. J. Reed and the like first crystally separated the trace ingredient from the liver and named as α-lipoic acid, and then the structure was established by synthesis. The α-lipoic acid physiologically acts as coenzyme closely related to thiamine in a complete oxidative decarboxylation system of pyruvic acid or alpha-ketoglutaric acid. The α-lipoic acid is recognized as an essential substance in metabolism, but is not known whether it is necessary for food intake of higher animals, and lipoic acid deficiency experiments in animals have not yet been completely successful. Recently, the α-lipoic acid and dihydrolipoic acid (DHLA) which is a reduced form thereof are known to have a function as an antioxidant, and it is reported that the α-lipoic acid and the DHLA react with reactive oxygen species such as peroxide radicals, hydroxyl radicals, peroxy radicals and singlet oxygen. Further, it is reported that the α-lipoic acid and the DHLA have a function of protecting cell membrane by reacting with vitamin C or glutathione and effectively acts on oxidative stress in the case of administrating α-lipoic acid.

N-acetylcysteine has been developed before about 50 years and mainly used as an expectorant, or a drug for inhibiting oxidative stress used in the treatment of poisoning of acetaminophen which is an analgesic. It is known that the N-acetylcysteine directly reacts with peroxide, hydrogen peroxide, hydroxyl radical, and the like to reduce oxidative stress, or has an antioxidant effect by providing cysteine which is a raw material of glutathione biosynthesis to indirectly increase glutathione synthesis. The N-acetylcysteine has been known as safe drugs without causing side effects while being used for decades in various clinical fields such as treatment of acute respiratory distress syndromes, treatment of acute lung oxygen toxicity and prevention of acute renal failure by contrast agents.

Meanwhile, the inventors verified that the α-lipoic acid had a pronounced appetite suppressant effect in mice (Kim MS et al., Nat Med. 2004) and the N-acetylcysteine had significant effects of inhibiting a weight gain and improving insulin resistance in rats (Korean Patent Registration No. 10-2003-0030302). However, during single administration of each ingredient, the effects are excellent only at a high concentration and a risk of side effects due to the drug is present. Therefore, the inventors verified that in the case of merge administration of α-lipoic acid and N-acetylcysteine, a synergistic effect was shown compared with the single administration of each ingredient, and thus a significantly excellent anti-obesity effect was shown even at a low concentration without causing toxicity in vivo, and thereafter, completed the present invention.

### [Disclosure]

### [Technical Problem]

The present invention is directed to provide a composition for preventing or treating obesity, containing α-lipoic acid and N-acetylcysteine as active ingredients.

The present invention is also directed to provide a food composition for preventing or improving obesity, containing α-lipoic acid and N-acetylcysteine as active ingredients.

The present invention is also directed to provide a method for preventing or treating obesity including administrating a composition containing α-lipoic acid and N-acetylcysteine as active ingredients to a subject.

### [Technical Solution]

One aspect of the present invention provides a composition for preventing or treating obesity, containing α-lipoic acid and N-acetylcysteine as active ingredients.

Another aspect of the present invention provides a food composition for preventing or improving obesity, containing α-lipoic acid and N-acetylcysteine as active ingredients.

Yet another aspect of the present invention provides a method for preventing or treating obesity including administrating a composition containing α-lipoic acid and N-acetylcysteine as active ingredients to a subject.

### [Advantageous Effects]

According to the present invention, the mixed composition of α-lipoic acid and N-acetylcysteine of the present invention can reduce a risk of side effects due to drugs with synergistic effects due to merge administration of two drugs, and can be usefully used for preventing or treating obesity with excellent effects of inhibiting a weight gain compared to each single composition containing α-lipoic acid or N-acetylcysteine.

### [Description of Drawings]

FIG. 1 is a diagram illustrating an effect of inhibiting a weight gain, in the case of single-administrating α-lipoic acid or N-acetylcysteine.
FIG. 2 is a diagram illustrating comparing effects of inhibiting a weight gain in the case of single-administrating α-lipoic acid or N-acetylcysteine and in the case of merge-administrating α-lipoic acid and N-acetylcysteine.

### [Modes of the Invention]

The present invention provides a composition for preventing or treating obesity, containing α-lipoic acid and N-acetylcysteine as active ingredients.

Further, the present invention provides a method for preventing or treating obesity including administrating a composition containing α-lipoic acid and N-acetylcysteine as active ingredients to a subject.

The composition includes a pharmaceutical composition or a food composition.

The α-lipoic acid or the N-acetylcysteine of the present invention may be synthesized by general organic synthesis methods or used by purchasing commercial reagents.

The α-lipoic acid contained in the composition of the present invention is not limited thereto, but is racemate or an R-isomer and preferably the R-isomer.

In the α-lipoic acid or the N-acetylcysteine of the present invention, since in the case of administrating the two ingredients in combination, an effect of inhibiting a weight gain is significantly prominent compared to a case of administrating each ingredient alone, the α-lipoic acid or the N-acetylcysteine of the present invention may be usefully used for preventing or treating obesity. When the α-lipoic acid having an effect of inhibiting appetite and the N-acetylcysteine increasing energy metabolism are administrated in combination compared to the case of independently administrating the drugs, the drugs have a synergetic effect which is a larger effect than an additive effect of adding an effect of each drug. Such a synergetic effect may have a strong weight reduction effect while decreasing side effects due to administration of a single drug at a high concentration.

The composition of the present invention may further include carriers, excipients, and diluents which are suitable and generally used for preparation of the pharmaceutical composition. Further, the composition of the present invention may be formulated and used in forms, such as an oral formulation such as a powder, a granule, a tablet, a capsule, a suspension, an emulsion, syrup, and an aerosol, an external preparation, a suppository, and a sterile injection solution according to a general method. It is preferred that suitable formulations which are known in the art use formulations disclosed in the document (Remington's Pharmaceutical Science, recently, Mack Publishing Company, Easton PA). The carrier, the excipient, and the diluent which may be included in the composition may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, and the like. When the composition is formulated, the formulation may be prepared by using a diluent or an excipient, such as filler, an extender, a binder, a wetting agent, a disintegrating agent, and a surfactant, which are generally used. A solid formulation for oral administration includes a tablet, a pill, a powder, a granule, a capsule, or the like, and the solid formulation may be prepared by mixing, with the composition, at least one excipient, for example, starch, calcium carbonate, sucrose or lactose, gelatin, and the like. Further, lubricants such as magnesium stearate and talc may be used in addition to simple excipients. A liquid formulation for oral administration may use a suspension, a solution, an emulsion, a syrup, and the like, and may include various excipients, for example, a wetting agent, a sweetener, an aromatic agent, a preserving agent, and the like in addition to water and liquid paraffin as simple diluents which are commonly used. A formulation for parenteral administration includes a sterile aqueous solution, a non-aqueous solution, a suspension, an emulsion, a lyophilizing agent, and a suppository. As the non-aqueous solution and the suspension, propylene glycol, polyethylene glycol, vegetable oil such as olive oil, injectable ester such as ethyl oleate, and the like may be used. As a matter of the suppository, witepsol, macrogol, tween 61, cacao butter, laurin butter, glycerogelatin, and the like may be used.

The term "administration" used in the present invention means providing a predetermined composition of the present invention to a subject by any suitable method.

A preferable dose of the pharmaceutical composition of the present invention varies according to a state and a weight of the subject, the degree of the disease, a drug form, and administration route and period, but may be properly selected by those skilled in the art. For the preferable effect, a mixture of the α-lipoic acid and the N-acetylcysteine of the present invention may be administrated with an amount of 0.1 mg/kg to 1000 mg/kg a day and administrated once or several times a day.

The pharmaceutical composition of the present invention may be administrated to the subject by various routes. All methods of administration may be expected, and for example, may be administered by oral, rectal or intravenous, intramuscular, subcutaneous, intrauterine dura mater, or cerebravascular injection.

The composition of the present invention may be used alone or in combination with methods using surgery, radiation therapy, hormone therapy, chemotherapy, and biological response modifiers for preventing or treating obesity.

In the present invention, the "health functional foods" mean foods having biomodulatory functions such as prevention or improvement of diseases, bio-defense, immunity, convalescent restoration, and aging suppression, and needs to be harmless to the human body when taken in the long term.

The composition of the present invention may be added to the health functional foods for the purpose of preventing or improving obesity. In the case where the α-lipoic acid and the N-acetylcysteine of the present invention are used as food additives, the α-lipoic acid and the N-acetylcysteine are added as it is or may be used with other foods or food ingredients, and may be suitably used according to general methods. The mixed amount of the active ingredients may be suitably determined according to the purpose of use (prevention, health, or therapeutic treatment). Generally, in preparation of foods or beverages, the α-lipoic acid and the N-acetylcysteine of the present invention is added with an amount of 15 wt% or less, preferably, 10 wt% or less with respect to a raw material. However, in the case of long-term administration for health and hygiene or health control, the amount may be the range or less. Since there is no problem in terms of safety, the active ingredients may be used with the amount in the range or more.

The kind of food is not particularly limited. Examples of the foods which may be added with the materials include meat, sausages, bread, chocolate, candies, snacks, cookies, pizza, Ramen, other noodles, gums, dairy products including ice cream, various soups, beverages, tea, drinks, alcohol drinks, vitamin complex, and the like, and include all health foods in the accepted meaning.

The health beverage composition of the present invention may include various flavors, natural carbohydrates, or the like as an additional ingredient, like general beverages. The natural carbohydrates described above may use monosaccharides such as glucose and fructose, disaccharides such as maltose and sucrose, natural sweeteners such as dextrin and cyclodextrin, synthetic sweeteners such as saccharin and aspartame, or the like. A ratio of the natural carbohydrate may be generally about 0.01 to 10 g and preferably about 0.01 to 0.1 g per 100 ml of the composition of the present invention.

Besides, the composition of the present invention may contain various nutrients, vitamins, electrolytes, flavoring agents, coloring agents, pectic acid and salt thereof, alginic acid and salt thereof, organic acid, a protective colloidal thickener, a pH adjusting agent, a stabilizer, a preservative, glycerin, alcohol, a carbonic acid agent used in a carbonated drink, or the like. Besides, the composition of the present invention may include pulps for preparing natural fruit juices, fruit juice beverages, and vegetable beverages. The ingredients may be used independently or in combination. Although the ratio of these additives is not critical, generally, the ratio is selected in a range of 0.01 to 0.1 part by weight per 100 parts by weight of the composition of the present invention.

Hereinafter, for helping in understanding of the present invention, preferred Examples and Formulation Examples will be described. However, the following Experimental Examples and Formulation Examples are provided for more easily understanding the present invention and the contents of the present invention are not limited to Experimental Examples and Formulation Examples.

### Experimental Example 1. Verification of effect of inhibiting weight gain by merge administration of α-lipoic acid and N-acetylcysteine

In order to verify that an effect of inhibiting a weight gain is significantly excellent by merge administration of α-lipoic acid and N-acetylcysteine compared to single-administration of α-lipoic acid or N-acetylcysteine, the following experiment was performed.

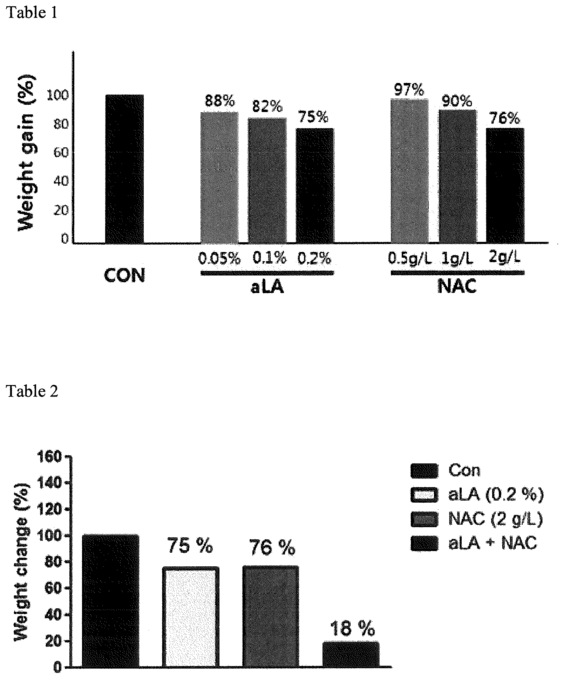

Particularly, 7-week-old C57/BL6J male mice were divided into a control group, an α-lipoic acid administrated group, an N-acetylcysteine administrated group, and an α-lipoic acid and N-acetylcysteine merge administrated group and took in high-fat diets configuring by 60% fat, 18% protein, and 22% carbohydrate without intake limitation. Simultaneously, in the α-lipoic acid administrated group, α-lipoic acid was added to the diet at concentrations of 0.05%, 0.1%, and 0.2% and taken for 3 weeks, and in the N-acetylcysteine administrated group, N-acetylcysteine was added to water at concentrations of 0.5 g/L, 1 g/L, and 2 g/L and taken as drinking water for 3 weeks, and then a weight gain degree was measured. The results are illustrated in FIG. 1.

Further, in mice belonging to the α-lipoic acid and N-acetylcysteine merge administrated group, a diet added with α-lipoic acid at a concentration of 0.2% and drinking water added with N-acetylcysteine at a concentration of 2 g/L were taken for 3 weeks and then a weight gain degree of each group was measured. The results are illustrated in FIG. 2.

As illustrated in FIGS. 1 and 2, it was verified that compared to an animal group of administrating α-lipoic acid or N-acetylcysteine, in an animal group of merge administrating α-lipoic acid and N-acetylcysteine, the weight gain was significantly inhibited and a synergetic effect between the drugs was present.

Hereinafter, Formulation Examples of pharmaceutical compositions for preventing or treating obesity and food compositions for preventing or improving obesity containing α-lipoic acid and N-acetylcysteine of the present invention as active ingredients will be described, but the present invention is not limited thereto and will be just described in detail.

### Preparation Example 1. Formulations of pharmaceutical composition

### 1-1. Preparation of powder

Mixture of α-lipoic acid and N-acetylcysteine 20 mg
Lactose 100 mg
Talc 10 mg
The ingredients were mixed and packed in an airtight bag to prepare the powder.

### 1-2. Preparation of tablet

Mixture of α-lipoic acid and N-acetylcysteine 10 mg
Corn starch 100 mg
Lactose 100 mg
Magnesium stearate 2 mg

The ingredients were mixed and tableted according to a general tablet preparing method to prepare the tablet.

### 1-3. Preparation of capsule

Mixture of α-lipoic acid and N-acetylcysteine 10 mg
Crystalline cellulose 3 mg
Lactose 14.8 mg
Magnesium stearate 0.2 mg

The ingredients were mixed and filled in a gelatin capsule according to a general capsule preparing method to prepare the capsule.

### 1-4. Preparation of injection

Mixture of α-lipoic acid and N-acetylcysteine 10 mg
Mannitol 180 mg
Sterile distilled water for injection 2974 mg
Na₂HPO₄.2H₂O 26 mg

The injection was prepared with the content of ingredients per 1 ampoule (2 ml) according to a general method of preparing the injection.

### 1-5. Preparation of solution

Mixture of α-lipoic acid and N-acetylcysteine 20 mg
Isomerized glucose 10 g
Mannitol 5 g
Purified water suitable amount

According to a general preparing method of the solution, respective ingredients were added and dissolved in purified water, added with a suitable amount of lemon flavoring, and mixed and then added with purified water so as to be adjusted to a total of 100 ml, and then filled in a dark amber bottle and sterilized to prepare the solution.

### Preparation Example 2. Preparation of food compositions

### 2-1. Preparation of health food

Mixture of α-lipoic acid and N-acetylcysteine 100 mg
Vitamin mixture suitable amount
Vitamin A acetate 70 µg
Vitamin E 1.0 mg
Vitamin B1 0.13 mg
Vitamin B2 0.15 mg
Vitamin B6 0.5 mg
Vitamin B12 0.2 µg
Vitamin C 10 mg
Biotin 10 µg
Nicotinic acid amide 1.7 mg
Folic acid 50 µg
Calcium pantothenate 0.5 mg
Mineral mixture suitable amount
Ferrous sulfate 1.75 mg
Zinc oxide 0.82 mg
Magnesium carbonate 25.3 mg
Monopotassium phosphate 15 mg
Dicalcium phosphate 55 mg
Potassium citrate 90 mg
Calcium carbonate 100 mg
Magnesium chloride 24.8 mg

The composition ratio of the mixture of vitamins and minerals was made by mixing ingredients relatively suitable for health foods as a preferable Example, but the mixed ratio may be randomly modified. According to a general method of preparing the health food, the ingredients were mixed to prepare granules and may be used for preparing the health food composition according to a general method.

### 2-2. Preparation of health beverage

Mixture of α-lipoic acid and N-acetylcysteine 100 mg
Vitamin C 15 g
Vitamin E (powder) 100 g
Iron lactate 19.75 g
Zinc oxide 3.5 g
Nicotinic acid amide 3.5 g
Vitamin A 0.2 g
Vitamin B1 0.25 g
Vitamin B2 0.3 g
Water Suitable amount

According to a general method of preparing health beverages, the ingredients were mixed, stirred and heated for about 1 hour at 85°C, a prepared solution was filtrated to be obtained in a sterilized container of 2 L, sterilized after sealing, and refrigerated, and then used for preparing the health beverage composition of the present invention.

The composition ratio was made by mixing the ingredients relatively suitable for a favorite beverage as preferable Example, but the mixed ratio may be randomly modified and implemented according to regional and national preference such as demand classes, demand countries, and purposes of use.

## Claims

1. A composition for use in a method of preventing or treating obesity, the composition comprising α-lipoic acid and N-acetylcysteine as active ingredients.

2. The composition for use of claim 1, wherein the α-lipoic acid is a racemate or an R-isomer.

3. The composition for use of claim 1 or 2, wherein said composition is a food composition.

## Patentansprüche

1. Zusammensetzung zur Verwendung in einem Verfahren zur Prävention oder Behandlung von Fettleibigkeit, wobei die Zusammensetzung α-Liponsäure und N-Acetylcystein als aktive Inhaltstoffe umfasst.

2. Zusammensetzung zur Verwendung nach Anspruch 1, wobei die α-Liponsäure ein Razemat oder ein R-Isomer ist.

3. Zusammensetzung zur Verwendung nach Anspruch 1 oder 2, wobei die Zusammensetzung eine Lebensmittelzusammensetzung ist.

## Revendications

1. Composition pour une utilisation dans une méthode de prévention ou de traitement de l'obésité, la composition comprenant de l'acide α-lipoïque et de la N-acétylcystéine comme ingrédients actifs.

2. Composition pour une utilisation selon la revendication 1, dans laquelle l'acide α-lipoïque est un racémate ou un isomère R.

3. Composition pour une utilisation selon la revendication 1 ou 2, où la composition est une composition alimentaire.
